# EUROPÄISCHE PATENTSCHRIFT

(11) **EP 1 041 954 B1**
(45) Veröffentlichungstag und Bekanntmachung des Hinweises auf die Patenterteilung: **30.06.2004**
(21) Anmeldenummer: 98965263.1
(22) Anmeldetag: 14.12.1998
(51) Int. Cl.: A61K 7/06

(54) **HAARBEHANDLUNGSMITTEL**
HAIR TREATMENT PRODUCTS
PRODUITS DE TRAITEMENT CAPILLAIRE

(30) Priorität: 23.12.1997 DE 19757508
(43) Veröffentlichungstag der Anmeldung: 11.10.2000
(73) Patentinhaber: Henkel Kommanditgesellschaft auf Aktien, 40589 Düsseldorf-Holthausen (DE)
(72) Erfinder: HÖFFKES, Horst, D-40595 Düsseldorf (DE); HOLLENBERG, Detlef, D-40699 Erkrath (DE); BERNECKER, Ullrich, D-52393 Hürtgenwald (DE)
(86) Internationale Anmeldenummer: PCT/EP1998/008200
(87) Internationale Veröffentlichungsnummer: WO 1999/033433

(56) Entgegenhaltungen:
- WO-A-95/31173
- DE-A- 4 134 137
- ES-A- 2 106 690
- US-A- 4 861 593
- US-A- 4 904 471
- US-A- 5 407 675
- US-A- 5 539 137
- US-A- 5 695 748
- PATENT ABSTRACTS OF JAPAN vol. 012, no. 047 (C-475), 12. Februar 1988 & JP 62 192312 A (KANEBO LTD), 22. August 1987
- PATENT ABSTRACTS OF JAPAN vol. 012, no. 283 (C-518), 3. August 1988 & JP 63 060916 A (SUNSTAR INC), 17. März 1988
- PATENT ABSTRACTS OF JAPAN vol. 097, no. 012, 25. Dezember 1997 & JP 09 208435 A (RIARU KAGAKU KK;YAMAKAWA BOEKI KK), 12. August 1997
- PATENT ABSTRACTS OF JAPAN vol. 098, no. 012, 31. Oktober 1998 & JP 10 194941 A (KAO CORP), 28. Juli 1998
- PATENT ABSTRACTS OF JAPAN vol. 097, no. 011, 28. November 1997 & JP 09 194340 A (POLA CHEM IND INC), 29. Juli 1997
- PATENT ABSTRACTS OF JAPAN vol. 097, no. 011, 28. November 1997 & JP 09 194339 A (POLA CHEM IND INC), 29. Juli 1997
- PATENT ABSTRACTS OF JAPAN vol. 006, no. 127 (C-113), 13. Juli 1982 & JP 57 053401 A (POLA CHEM IND INC), 30. März 1982

## Beschreibung

Die Erfindung betrifft Haarbehandlungsmittel mit glanzverbessernder Wirkung enthaltend physiologisch verträgliche Substanzen mit einem Brechungsindex oberhalb von 1,48 sowie die Verwendung ausgewählter Substanzen mit einem Brechungsindex oberhalb von 1,48 in Haarbehandlungsmitteln zur glanzverbessernden Wirkung.

Gesundes Haar weist einen natürlichen Glanz auf. Aufgrund von Umwelteinflüssen wie beispielsweise Sonneneinstrahlung kann es jedoch zu einer Veränderung der Haaroberfläche und damit einhergehend zum Verlust des natürlichen Glanzes kommen. Auch kosmetische Maßnahmen wie Dauerwelle, Färben, Blondieren und/ oder Tönen sowie mechanische Einflüsse beim Kämmen und Bürsten können die Haarstruktur beeinflussen und zu einer Minderung des Glanzes führen.

Da ein natürlicher Glanz der Haare erwünscht ist, hat es nicht an Versuchen gemangelt, diesen zu erhalten bzw. wiederherzustellen. Es ist bereits bekannt, daß eine glättende Behandlung der Haaroberfläche, wie dies beispielsweise durch Spülungen und/ oder Kuren erreicht werden soll, zu einer Erhöhung des gerichtet reflektierten Lichtanteils führt und damit den Glanz erhöht. In der Literatur sind insbesondere Silikonöle als oberflächenglättend zum Einsatz in Haarbehandlungsmitteln bereits beschrieben (Siehe insb. WO 95/31173).

Silikonöle können zu einer kosmetisch nicht erwünschten Beschwerung der Haare führen. Es besteht daher ständig Bedarf nach weiteren Substanzen, die diese Nachteile nicht aufweisen.

Überraschenderweise wurde nun gefunden, daß durch Verwendung von bestimmten Substanzen mit einem Brechungsindex oberhalb von 1,48 in Haarbehandlungsmittel eine glanzverbessernde Wirkung auf dem Haar hervorgerufen werden kann.

Gegenstand der vorliegenden Erfindung sind daher Haarbehandlungsmittel enthaltend physiologisch verträgliche Substanzen mit einem Brechungsindex größer 1,48, vorzugsweise größer 1,50 und insbesondere größer 1,55 in Kombination mit Kokosfettalkohol. Im Rahmen der vorliegenden Erfindung handelt es sich um den Brechungsindex bestimmt bei 20 °C mittels der Na_{D}-Linie. Ein weiterer Gegenstand der Erfindung betrifft die Verwendung von physiologisch verträglichen Verbindungen mit einem Brechungsindex größer 1,48, vorzugsweise größer 1,50 und insbesondere größer 1,55 in Haarbehandlungsmitteln.

In einer bevorzugten Ausführungsform handelt es sich um Verbindungen, die bei Temperaturen von 20 bis 60, vorzugsweise 20 bis 35, °C und Normaldruck (1013 mbar) in flüssiger Form vorliegen.

Typische erfindungsgemäß einzusetzende Substanzen mit einem Brechungsindex oberhalb von 1,48 sind beispielsweise folgende Verbindungen (in Klammern ist jeweils der Brechungsindex angegeben): 2-Aminoacetophenon (n_{D}²⁰ =1,6160), o-Anisaldehyd (n_{D}²⁰ = 1,5600), m-Anisaldehyd (n_{D}²⁰ = 1,5530), p-Anisaldehyd (n_{D}²⁰ = 1,5730), N-Methylanthranilsäuremethylester (n_{D}²⁰ = 1,5839), Anthranilsäuremethylester (n_{D}²⁰ = 1,5810), 1-Methylbenzimidazol (n_{D}²⁰ = 1,6013), Benzophenoncarbonsäuremethylester (n_{D}²⁰ = 1,5910), 3-Chromanon (n_{D}²⁰ = 1,5460), trans-Zimtsäuremethylester (n_{D}²⁰ = 1,5766), 3,4-Dihydrocumarin (n_{D}²⁰ = 1,5563), Tropon (n_{D}²⁰ = 1,6172), cis-Isoeugenol (n_{D}²⁰ = 1,5726), trans-Isoeugenol (n_{D}²⁰ = 1,5784), 2-Furylphenylketon (n_{D}²⁰ = 1,6055) 1-Phenylimidazol (n_{D}²⁰ = 1,6025), Indan (n_{D}²⁰ = 1,5978), Indolin (n_{D}²⁰ = 1,5923), Isochinolin (n_{D}²⁰ = 1,6148), trans-Isosafrol (n_{D}²⁰ = 1,5782), Jasminaldehyd (n_{D}²⁰ =1,5381), 1-Methylisochinolin (n_{D}²⁰ = 1,6095), Chinaldin (n_{D}²⁰ = 1,8116), 2-Methylchinolin. Eine weitere erfindungsgemäß einzusetzende Verbindung ist Hennaöl.

Bevorzugt werden p-Anisaldehyd, Anthranilsäuremethylester, Zimtsäuremethylester, Indolin und/ oder Isosafrol eingesetzt.

Die erfindungsgemäßen Mittel enthalten die Substanzen mit einem Brechungsindex größer 1,48 vorzugsweise in Mengen von 0,1 bis 10, insbesondere 0,5 bis 5 Gew.-%.

Die erfindungsgemäßen Wirkstoffe lassen sich mit dem Fachmann geläufigen Methoden in bekannte Formulierungen einarbeiten und bewirken nach der Anwendung auf dem Haar eine deutliche Verbesserung des Glanzes.

Neben der obligatorischen Substanz mit einem Brechungsindex oberhalb von 1,48 können die erfindungsgemäßen Haarbehandlungsmittel alle für solche Mittel üblichen Komponenten enthalten. Die Auswahl dieser weiteren Komponenten wird im wesentlichen durch die Art des Haarbehandlungsmittels bestimmt.

Bevorzugt sind Haarbehandlungsmittel, die zusätzlich Fettalkohole enthalten. Unter Fettalkoholen sind primäre aliphatische Alkohole der Formel (I) zu verstehen,

**R**^{**1**}**OH (I)**

in der R¹ für einen aliphatischen, linearen oder verzweigten Kohlenwasserstoffrest mit 6 bis 22 Kohlenstoffatomen, der gesättigt ist oder bis zu 3 Doppelbindungen enthalten kann, steht.

Typische Beispiele sind Capronalkohol, Caprylalkohol, 2-Ethylhexylalkohol, Caprinaikohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinyialkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind technische Fettalkohole mit 12 bis 18 Kohlenstoffatomen wie beispielsweise Palm-, Palmkern- oder Talgfettalkohol. Vorzugsweise enthalten die Mittel 0,5 bis 50, insbesondere 2 bis 20 Gew.-% Fettalkohol.

Neben den Fettalkoholen können vorzugsweise auch noch Tenside in den erfindungsgemäßen Haarbehandlungsmitteln enthalten sein. Es handelt sich dabei in einer bevorzugten Ausführungsform um kationische Tenside, Seifen und/ oder Di- sowie Polycarbonsäuren.

Als **kationische Tenside** kommen beispielsweise quartäre Ammoniumverbindungen, Amidoamine sowie quaternisierte Ester und Proteinhydrolysate in Frage.

**Di- und Polycarbonsäuren** im Sinne der vorliegenden Anmeldung sind beispielsweise Verbindungen der Formel **(II),** 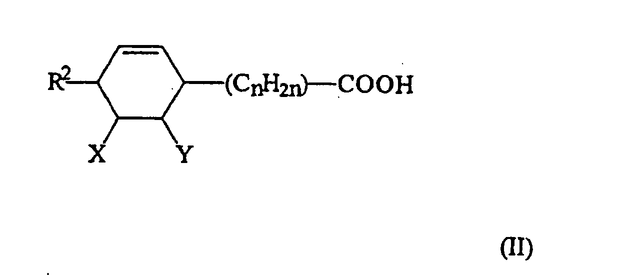 in der R² steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (II), die zusätzlich noch 1 bis 3 Methyl- oder Ethylsubstituenten am Cyclohexenring tragen, Dicarbonsäuren, die aus den Dicarbonsäuren gemäß Formel (II) formal durch Anlagerung eines Moleküls Wasser an die Doppelbindung im Cyclohexenring entstehen, oder einem physiologisch verträglichen Salz einer dieser Säuren. Ein Herstellungsverfahren für Dicarbonsäuren ist beispielsweise der US-Patentschrift 3,753,968 zu entnehmen.

Es sind solche Dicarbonsäuren der Formel (II) bevorzugt, bei denen R² steht für eine lineare oder methylverzweigte, gesättigte Alkylgruppe mit 4 bis 8 Kohlenstoffatomen und n für eine Zahl von 6 bis 10.

Erfindungsgemäß einsetzbar neben den, bevorzugten, Dicarbonsäuren gemäß Formel (II) sind auch solche Dicarbonsäuren, die sich von den Verbindungen gemäß Formel (II) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid® 1550 und Westvaco Diacid® 1595 (Hersteller: Westvaco) erhältlich.

Neben den Dicarbonsäuren selbst können auch deren physiologisch verträgliche Salze erfindungsgemäß eingesetzt werden. Beispiele für solche Salze sind die Alkali-, Erdalkali-, Ammonium-, die Mono-, Di- und Trimethyl-, -ethyl- und -hydroxyethyl-Ammoniumsalze sowie das Zinksalz. Die Natrium-, Kalium- und Ammoniumsalze sind bevorzugte Salze. Weiterhin kann es aus Formulierungsgründen bevorzugt sein, die Dicarbonsäure aus den wasserlöslichen Vertretern, insbesondere den wasserlöslichen Salzen auszuwählen. Das Kaliumsalz der 5- und 6-Carboxy-4-hexyl-2-cyclohexen-1-octansäure, das als wäßrige Lösung unter der Bezeichnung Westvaco Diacid® H-240 kommerziell erhältlich ist, hat sich als besonders geeignet erwiesen.

Unter Dicarbonsäuren im Sinne der vorliegenden Anmeldung sind jedoch auch Alkylsowie Alkenylbemsteinsäuren mit 1 bis 18 C-Atomen in der Alkylkette, zu verstehen, sowie deren physiologisch verträgliche Salze.

Weiterhin bevorzugt werden zusätzlich **wasserlösliche Polymere** eingesetzt. Dabei kann es sich im Sinne der vorliegenden Erfindung sowohl um nichtionische, kationische, anionische und/ oder amphotere Polymere handeln. Bevorzugte Polymere sind die nichtionischen sowie die kationischen Polymere wie beispielsweise Polyvinylpyrrolidon, Vinylpyrrolidon/Vinylacetat-Copolymere und Celluloseether, Stärke, Cyclodextrine, Guarderivate, Cellulosederivate sowie die unter den Handelsnamen Cosmedia Guar® und Polymer JR® erhältlichen Produkte.

Eine weitere Klasse fakultativer Komponenten stellen die nichtionogenen Tenside dar.

Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bei diesen Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den nichtionogenen Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Alkylpolyglykoside gemäß der Formel RO-(Z)_{X}, in der R steht für einen Alkylrest mit 8 bis 22 Kohlenstoffatomen, Z für einen Mono- oder Oligosaccharid und x für eine Zahl von 1,1 bis 5, sind besonders bevorzugte nichtionogene Tenside. Diese Verbindungen sind durch die folgenden Parameter gekennzeichnet.

Der Alkylrest R enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Unter Haarbehandlungsmittel fallen auch jene Mittel zum Färben und Tönen der Haare, solche Mittel enthalten entweder sogenannte "direktziehende" Farbstoffe und/oder Vorprodukte für Oxidationsfarbstoffe.

Übliche direktziehende Farbstoffe darunter sind zu verstehen, z. B. Verbindungen aus der Gruppe der Nitrophenylendiamine, Nitroaminophenole, Azofarbstoffe, Anthrachinone oder Indophenole, wie beispielsweise unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen, sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol und 4-N-Ethyl-1,4-bis-(2'-hydroxyethylamino)-2-nitrobenzol-hydrochlorid in einer Menge von 0,01 bis 20 Gew.-%, bezogen auf das gesamte Oxidationshaarfärbemittel. 4-Amino-2-nitro-diphenylamin-2'-carbonsäure, 6-Nitro-1,2,3,4-tetrahydrochinoxalin und HC Red BN sind erfindungsgemäß besonders bevorzugte direktziehende Farbstoffe.

In einer bevorzugte Ausführungsform können auch Indole und/oder Indoline enthalten sein. Im Sinne der vorliegenden Erfindung besonders bevorzugt sind 6-Hydroxyindol, N-Methyl-6-Hydroxyindol, N-Ethyl-6-Hydroxyindol, N-Propyl-6-Hydroxyindol, N-Butyl-6-Hydroxyindol, 4-Hydroxyindol, N-Methyl-4-Hydroxyindol, N-Ethyl-4-Hydroxyindol, N-Propyl-4-Hydroxyindol, N-Butyl-4-Hydroxyindol, 5,6-Dihydroxyindol, N-Methyl-5,6-Dihydroxyindol, N-Ethyl-5,6-Dihydroxyindol, N-Propyl-5,6-Dihydroxyindol, N-Butyl-5,6-Dihydroxyindol sowie 5,6-Dihydroxyindolin, N-Methyl-5,6-Dihydroxyindolin, N-Ethyl-5,6-Dihydroxyindolin, N-Propyl-5,6-Dihydroxyindolin und N-Butyl-5,6-Dihydroxy-indolin.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Als Vorprodukte für Oxidationsfarbstoffe enthalten Haarfärbemittel sogenannte Entwickler- und Kupplerkomponenten. Die Entwicklerkomponenten bilden unter dem Einfluß von Oxidationsmitteln oder Luftsauerstoff untereinander oder unter Kupplung mit einer oder mehreren Kupplerkomponenten die eigentlichen Farbstoffe aus.

Als **Entwicklerkomponenten** werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind beispielsweise p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 2-Aminomethyl-4-aminophenol und 4-Amino-3-methylphenol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol und 2-Methylresorcin.

Die Art der erfindungsgemäßen kosmetischen Mittel unterliegt keinen Beschränkungen. Die erfindungsgemäßen Mittel können sowohl auf dem Haar verbleiben (leave-on), als auch nach einer Einwirkzeit von wenigen Sekunden bis Minuten wieder ausgespült werden (rinse-off). Bevorzugt ist der Einsatz der erfindungsgemäßen Wirkstoffkombination in den sogenannten *rinse-off* Produkten. Beispiele für erfindungsgemäße Mittel sind Shampoos, Spülungen, Kuren, Konditioniermittel, Tönungsmittel, Färbemittel, Dauerwellmittel, Fixiermittel, Haarsprays und Fönwellen.

Weitere Komponenten dieser Mittel können dann beispielsweise sein:
- anionische Tenside, wie beispielsweise Fettalkylsulfate und -ethersulfate sowie Alkylethercarbonsäuren,
- zwitterionische Tenside, wie beispielsweise Betaine,
- ampholytische Tenside,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle und Dimethylisosorbid,
- Lösungsvermittler, wie Ethanol, Isopropanol, Ethylenglykol, Propylenglykol, Glycerin und Diethylenglykol,
- Farbstoffe,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Wachse, wie Bienenwachs und Montanwachs,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- Reduktionsmittel wie z. B. Thioglykolsäure und deren Derivate, Thiomilchsäure, Cysteamin, Thioäpfelsäure und α-Mercaptoethansulfonsäure,

Die erfindungsgemäßen Mittel können beispielsweise als wäßrige, alkoholische oder wäßrig-alkoholische Lösungen, Cremes, Gele oder Emulsionen formuliert sein.

Es hat sich gezeigt, daß praktisch unabhängig von der Art des Haarbehandlungsmittels bestimmte weitere Komponenten die erfindungsgemäße Wirkstoffkombination in ihrer Wirkung besonders vorteilhaft ergänzen.
Als besonders vorteilhaft haben sich Haarbehandlungsmittel erwiesen, die neben der erfindungsgemäßen Wirkstoffkombination **Acyllactylate** der allgemeinen Formel (III) 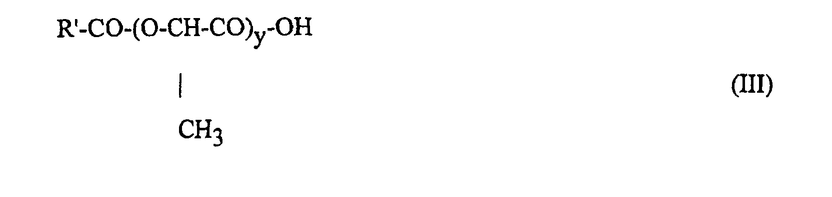 in der R' für eine lineare oder verzweigte, gesättigte oder ungesättigte Alkyl- oder Alkenylgruppe mit 6 bis 22 Kohlenstoffatomen steht und y für eine Zahl von 1 bis 5, enthalten.

Acyllactylate, bei denen R' für eine, insbesondere gesättigte, lineare oder methylverzweigte Alkylgruppe mit 12 bis 18 Kohlenstoffatomen steht und y für eine Zahl von 1 bis 3, sind besonders bevorzugt.

Solche Acyllactylate sind im Handel unter dem Warenzeichen Pationic® erhältlich.

Ein Natriumisostearoyllactyllactylat, das unter der Bezeichnung Pationic®ISL vertrieben wird, ist ein besonders bevorzugtes Acyllactylat.

Weiterhin bevorzugt in erfindungsgemäß verwendeten haarkosmetischen Mitteln einzusetzende Substanzen sind beispielsweise:
- Antischuppenwirkstoffe wie Piroctone Olamine und Zink Omadine,
- haarkonditionierende Verbindungen wie Phospholipide, beispielsweise Sojalecithin, Ei-Lecitin und Kephaline, sowie Silikonöle,
- Oxidationsmittel wie Wasserstoffperoxid, Kaliumbromat und Natriumbromat.

Die folgenden Beispiele sollen den Gegenstand der Erfindung näher erläutern.

### Beispiele

### Tönungslotion (Kupfer)

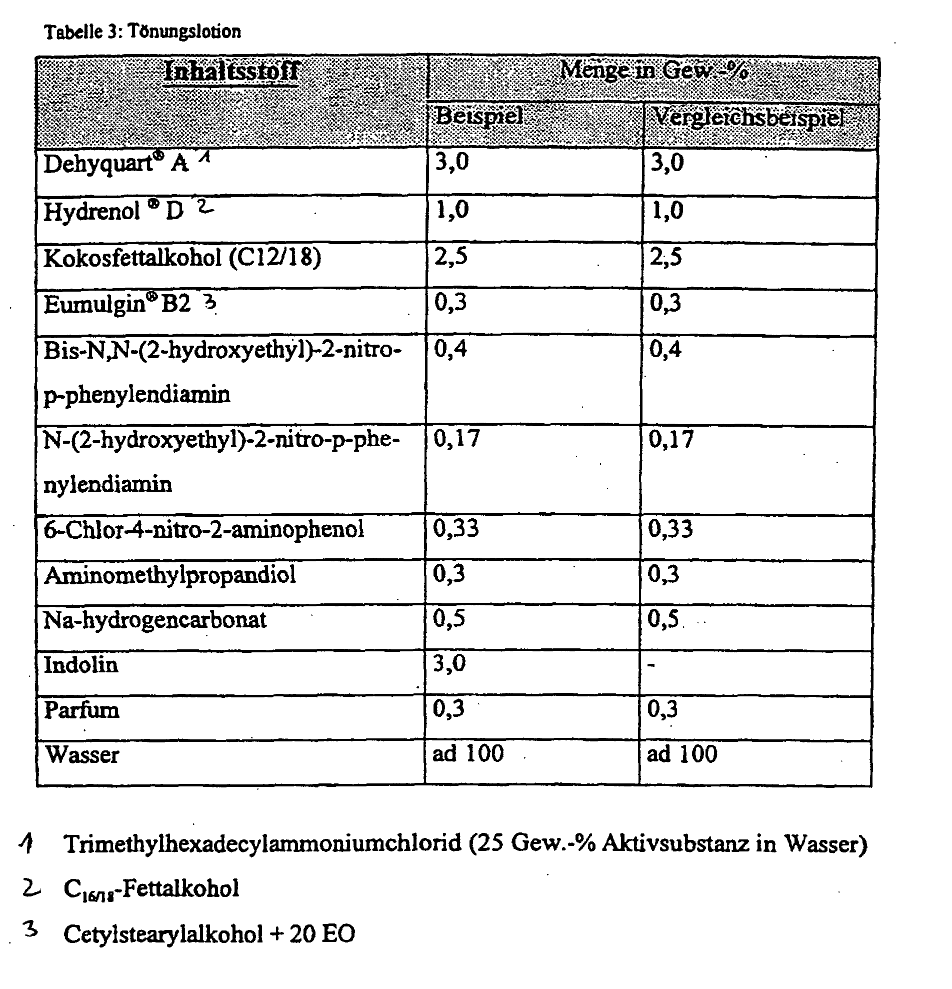

Die Lotionen gemäß Beispiel 1 bis 3 (**B1, B2, B3**) und Vergleichsbeispiel 1 bis 3 (**V1, V2, V3**) wurden jeweils halbseitig auf rotblondem Haar aufgetragen. Anschließend wurde die Glanzwirkung der Haare subjektiv bewertet.

Die erfindungsgemäßen Rezepturen (B1, B2, B3) wurden deutlich besser bewertet als die Vergleichsrezepturen (V1, V2, V3).

## Patentansprüche

1. Haarbehandlungsmittel enthaltend physiologisch verträgliche Substanzen mit einem Brechungsindex oberhalb von 1,48, **dadurch gekennzeichnet, daß** die Substanzen ausgewählt sind aus der Gruppe, die gebildet wird von 2-Aminoacetophenon, o-Anisaldehyd, m-Anisaldehyd, p-Anisaldehyd, N-Methylanthranilsäuremethylester, Anthranilsäuremethylester, 1-Methylbenzimidazol, Benzophenoncarbonsäuremethylester, 3-Chromanon, trans-Zimtsäuremethylester, 3,4-Dihydrocumarin, Tropon, cis-Isoeugenol, trans-Isoeugenol, 2-Furylphenylketon, 1-Phenylimidazol, Indan, Indolin, Isochinolin, trans-Isosafrol, Jasminaldehyd, 1-Methylisochinolin, Chinaldin, 2-Methylchinolin sowie Hennaöl, und das Haarbehandlungsmittel weiterhin Kokosfettalkohol enthält.

2. Haarbehandlungsmittel nach Anspruch 1, **dadurch gekennzeichnet, daß** die Substanzen in Mengen von 0,1 bis 10 Gew.-% enthalten sind.

3. Haarbehandlungsmittel nach einem der Ansprüche 1 bis 2, **dadurch gekennzeichnet, daß** es 0,5 bis 50 Gew.-% Kokosfettalkohol enthält.

4. Haarbehandlungsmittel nach einem der Ansprüche 1 bis 3, **dadurch gekennzeichnet, daß** es Dicarbonsäuren und/ oder deren physiologisch verträgliche Salze enthält.

5. Haarbehandlungsmittel nach einem der Ansprüche 1 bis 4, **dadurch gekennzeichnet, daß** es Seifen und/ oder quartäre Ammoniumverbindungen enthält.

6. Haarbehandlungsmittel nach einem der Ansprüche 1 bis 5, **dadurch gekennzeichnet, daß** es kationische und/ oder nichtionische Polymere enthält.

7. Verwendung eines Mittels nach einem der Ansprüche 1 bis 6 zur Glanzverbesserung der Haare.

## Claims

1. Hair treatment preparation containing physiologically safe substances with a refractive index above 1.48, **characterized in that** the substances are selected from the group consisting of 2-aminoacetophenone, o-anisaldehyde, m-anisaldehyde, p-anisaldehyde, N-methyl anthranilic acid methyl ester, anthranilic acid methyl ester, 1-methylbenzimidazole, benzophenone carboxylic acid methyl ester, 3-chromanone, trans-cinnamic acid methyl ester, 3,4-dihydrocoumarin, tropon, cis-isoeugenol, trans-isoeugenol, 2-furylphenyl ketone, 1-phenyl imidazole, indane, indoline, isoquinoline, trans-isosafrol, jasmine aldehyde, 1-methyl isoquinoline, quinaldine, 2-methyl quinoline and henna oil and the hair treatment preparation also contains coconut oil fatty alcohol.

2. Hair treatment preparation as claimed in claim 1, **characterized in that** the substances are present in quantities of 0.1 to 10% by weight.

3. Hair treatment preparation as claimed in claim 1 or 2, **characterized in that** it contains 0.5 to 50% by weight coconut oil fatty alcohol.

4. Hair treatment preparation as claimed in any of claims 1 to 3, **characterized in that** it contains dicarboxylic acids and/or physiologically safe salts thereof.

5. Hair treatment preparation as claimed in any of claims 1 to 4, **characterized in that** it contains soaps and/or quaternary ammonium compounds.

6. Hair treatment preparation as claimed in any of claims 1 to 5, **characterized in that** it contains cationic and/or nonionic polymers.

7. The use of the preparation claimed in any of claims 1 to 6 for improving the lustre of hair.

## Revendications

1. Agent de traitement des cheveux contenant des substances physiologiquement acceptables avec un indice de réfraction supérieur à 1,48, **caractérisé en ce que** les substances sont choisies dans le groupe constitué par la 2-aminoacétophénone, le o-anisaldéhyde, le m-anisaldéhyde, le p-anisaldéhyde, l'ester méthylique de l'acide N-méthylanthranilique, l'ester méthylique de l'acide anthranilique, le 1-méthylbenzimidazole, l'ester méthylique de l'acide benzophénonecarboxylique, la 3-chromanone, l'ester méthylique de l'acide trans-cinnamique, la 3,4-dihydrocoumarine, la tropone, le cis-isoeugénol, le trans-isoeugénol, la 2-furylphénylcétone, le 1-phénylimidazole, l'indane, l'indoline, l'isoquinoléine, le trans-isosafrol, le jasminaldéhyde, la 1-méthylisoquinoléine, la quinaldine, la 2-méthylquinoléine ainsi que l'huile de henné, et **en ce que** l'agent de traitement des cheveux contient en outre un alcool gras de coprah.

2. Agent de traitement des cheveux selon la revendication 1, **caractérisé en ce que** les substances sont contenues à des quantités de 0,1 à 10% en poids.

3. Agent de traitement des cheveux selon l'une des revendications 1 à 2, **caractérisé en ce qu'**il contient de 0,5 à 50% en poids d'alcool gras de coprah.

4. Agent de traitement des cheveux selon l'une des revendications 1 à 3, **caractérisé en ce qu'**il contient des acides dicarboxyliques et/ou leurs sels physiologiquement acceptables.

5. Agent de traitement des cheveux selon l'une des revendications 1 à 4, **caractérisé en ce qu'**il contient des savons et/ou des composés d'ammonium quaternaire.

6. Agent de traitement des cheveux selon l'une des revendications 1 à 5, **caractérisé en ce qu'**il contient des polymères cationiques et/ou non ioniques.

7. Utilisation d'un agent selon l'une des revendications 1 à 6 pour améliorer l'éclat des cheveux.
